# EUROPEAN PATENT APPLICATION

(11) **EP 1 352 955 A2**
(43) Date of publication of application: **15.10.2003**
(21) Application number: 03252152.8
(22) Date of filing: 04.04.2003
(51) Int. Cl.: C12N 5/08, A61K 39/00, A61P 35/00, A61P 37/00

(54) **HLA matching donor-originating activated lymphocytes to be used in prevention/treatment of tumors, infectious diseases and autoimmune diseases, treatment method achieved by using the lymphocytes, formula having the lymphocytes as a main constitutuent thereof, method for manufacturing the formula and preparation kit to be used to prepare the formula**

(30) Priority: 08.04.2002 JP 2002104644; 18.02.2003 JP 2003039618
(71) Applicant: Lymphotec Inc., Tokyo (JP)
(72) Inventor: Kuroiwa, Yasuyuki, Hitachinaka-shi, Ibaraki (JP); Morio, Tomohiro, Tokyo 160-0015 (JP); Shimizu, Norio, Kitakoma-Gun, Yamanashi (JP); Sekine, Teruaki, Tokyo (JP)
(74) Representative: Rees, Alexander Ellison

(57) **Abstract**

According to the present invention, HLA matching donor-originating activated lymphocytes highly effective in the treatment of patients with tumors, viral infections and autoimmune diseases are prepared by collecting cells contained in peripheral blood originating from an HLA matching donor or peripheral blood originating from an HLA matching donor achieving micro-chimerism and by stimulating and propagating the cells thus collected with, for instance, interleukin 2 and anti-CD3 antibodies or the like. The HLA matching donor-originating activated lymphocytes thus prepared can be administered to an HLA matching patient with a tumor, a viral infection or an autoimmune disease for therapeutic or preventive purposes.

## Description

The present invention relates to HLA matching donor-originating activated lymphocytes to be used in the prevention and treatment of tumors, infectious diseases and autoimmune diseases, a treatment method achieved by using the lymphocytes, a formula having the lymphocytes as a main constituent thereof, a method for manufacturing the formula and a preparation kit to be used to prepare the formula. An object of the present invention is to enable more effective treatment and prevention of tumors such as cancer and of infectious diseases.

A great deal of interest has been focused on lymphocytes as a crucial biophylaxic means for supporting the immune system in recent years. T-lymphocytes, which play a particularly important role in achieving cell-mediated immunity, are classified as follows, based upon their monoclonal antibody reactivity. For instance, a T-lymphocyte manifesting reactivity to anti-CD3 antibodies (CD stands for "cluster of differentiation") is classified as a CD3 positive cell. To date, quantitatively and qualitatively significant research work has been conducted on T-lymphocytes, specifically with regard to the relationship between the antigens that are manifested and their functions.

In addition, Mr. Sekine, one of the inventors of the present invention, previously reported in Japanese Unexamined Patent Publication No. H 03-80076 that lymphocytes can be propagated by using solid-phase anti-CD3 antibodies (coated with lymphocytes) and interleukin 2, and that the autologous lymphocytes thus propagated achieve a significant antineoplastic effect. Numerous other researchers have also reported that lymphocytes originating from peripheral blood or the like can be propagated by using anti-CD3 antibodies or interleukin 2 and that the resulting autologous lymphocytes manifest an antineoplastic activity.

Furthermore, a donor leukocyte transfusion (hereafter simply referred to as a "DLT") and a donor lymphocyte infusion (hereafter simply referred to as a "DLI") are two well established treatment approaches adopted when leukemia recurs in a patient having undergone a bone marrow transplant, in which lymphocytes of the bone marrow donor are administered in large quantities. Also, the presence of micro-chimerism, whereby an immunological tolerance manifests between an HLA matching mother and child pair has been proved in recent years and, as a result, various types of research have been undertaken in order to take advantage of micro-chimerism to promptly induce a state of remission in the patient and thus enable a safer organ transplant. It is widely known that the success rate of sibling-to-sibling kidney transplant is very high when micro-chimerism is achieved between the siblings.
However, the research into T-lymphocytes and the induction of micro-chimerism mentioned above is still at an experimental stage and no applied technologies toward the realization of practical application of the research studies have been developed yet. In addition, a valid antineoplastic effect cannot always be assured, particularly in the case of cancer, simply by propagating harvested autologous lymphocytes. The relative difficulty in implementing the DLT treatment is another problem that must be addressed.

Leukocytes are classified into numerous different "loci", based upon the leukocyte groups that the individual leukocytes belong to, i.e., HLAs (human leukocyte antigens). Among these loci there are loci DQ and DP as well the four loci A, B, C and DR which are crucial factors that must be examined with care in organ transplants. These loci are each constituted of two copies of genes, one originating from the father and the other originating from the mother (allelic genes) and each locus is highly polymorphic. For this reason, in a blood transfusion as referred to in the broader sense, including transplants (such as peripheral blood hepatocyte transplants) and DLTs, at least four pairs of HLA loci in the donor and the patient must achieve a match and the rate of concordance of 8 HLA molecules is a crucial factor.

While it is nearly impossible for the patient and the donor to achieve a perfect leukocyte HLA match under these circumstances, a bone marrow transplant or a blood transfusion is undertaken in reality as long as the main HLAs match. However, an HLA mismatch may cause a fatal side effect, i.e., a fatal post transfusion graft-vs-host disease (hereafter simply referred to as a "PT-GVHD") in the patient having undergone a bone marrow transplant or a DLT treatment.

In addition, there are many patients who cannot undergo bone marrow transplants or blood transfusions even if they have HLA matching donors, due to pre-procedure manifestations of organopathy and infectious diseases. While attempts have been made to treat tumors in cancer patients for whom bone marrow transplants are not an option by administering donor-originating lymphocytes, such attempts have not proved very effective.

As described above, the antineoplastic effect of autologous (patient-originating) lymphocytes or donor-originating lymphocytes which are used when a bone marrow transplant is not an option, is not always satisfactory, and their anti-viral effect and their effect on autoimmune diseases have not been clarified yet. There is another problem in that lymphocytes cannot be prepared in large quantities without placing a great onus on the lymphocyte donor. Furthermore, a DLI, which must be performed in conjunction with hematopoietic stem cell transplantation, imposes significant restrictions as a treatment option, in that a DLI cannot be performed on a patient who does not facilitate hematopoietic stem cell transplantation and in that since the DLI performed in conjunction with a stem cell transplant requires a high rate of HLA concordance, the chances of finding a matching donor becomes slim.

Accordingly, the inventors of the present invention have completed the present invention through concentrated research efforts toward the development of donor-originating lymphocytes achieving and high antineoplastic effect, anti-viral effect and anti-autoimmune disease effect and toward development of a method for preparing such lymphocytes.

The present invention relates to HLA matching donor-originating activated lymphocytes achieving high antineoplastic effect and antiviral effect and also proving highly effective in the treatment of autoimmune diseases, which are obtained by harvesting cells contained in the peripheral blood of an HLA matching donor or the peripheral blood originating from an HLA matching donor manifesting micro-chimerism with the patient and then by stimulating and propagating the cells with, for instance, interleukin 2 and anti-CD3 antibodies or the like, a method for manufacturing such activated lymphocytes, a method for treating an HLA matching patient with a tumor, a viral infection or an autoimmune disease by administering the activated lymphocytes, a formula having the lymphocytes as a main constituent thereof, a method for manufacturing the formula and a preparation kit to be used to manufacture the formula.

While specific details of the present invention are provided in the following explanation, the present invention is characterized in summary in that HLA matching donor-originating activated lymphocytes are prepared by harvesting cells contained in the peripheral blood of an HLA matching donor or an HLA matching donor manifesting micro-chimerism and by stimulating and propagating the cells thus collected.
The harvested cells may be stimulated and propagated through, for instance, polyclonal activation achieved by using interleukin 2 and anti-CD3 antibodies. Alternatively, activated lymphocytes with antigenic specificity can be obtained by deriving antigen specific T-lymphocytes or the like with an appropriate antigen or the like and then by using one of; CD3 antibodies, CD26 antibodies and a cytokine such as interleukin 2 or by using them in combination. In addition, after propagating and activating harvested HLA matching donor-originating lymphocytes, activated lymphocytes with antigenic specificity may be selected through screening or antigen specific activated lymphocytes may be derived with the use of and an appropriate antigen to prepare HLA matching donor-originating lymphocytes.

Or activated lymphocytes achieving antigenic specificity may be selected by screening the activated lymphocytes mentioned above or activated lymphocytes achieving antigenic specificity may be prepared for use by propagating and activating the lymphocytes through antigen stimulation. In such a case, antigen specific lymphocytes can be derived either from CD8 positive cells or CD4 positive cells. In addition to a refined antigen, an extract from a cancer cell or a virus, the cancer cell itself or the virus itself or any antigenic material manifesting cross reactivity to the cancer cell or the virus may be used for these purposes.

In a specific mode of the present invention, HLA matching donor-originating activated lymphocytes are used to prevent or treat a tumor, an infectious disease and autoimmune disease.

In addition, according to the present invention, a formula containing the HLA matching donor-originating activated lymphocytes is used to prevent or treat tumors, various infectious diseases and autoimmune diseases.

Furthermore, the present invention provides a method of treatment achieved by using the HLA matching donor-originating activated lymphocytes or the formula containing the HLA matching donor-originating activated lymphocytes, which may be adopted to prevent or treat tumors, infectious diseases and autoimmune diseases.

The present invention further provides a preparation kit to be used to prepare activated lymphocytes or a formula achieved by combining HLA matching donor-originating lymphocytes with at least interleukin 2, which activates the lymphocytes. In addition, the present invention provides a preparation kit to be used to prepare activated lymphocytes or a formula achieved by combining HLA matching donor-originating lymphocytes with at least anti-CD3 antibodies, which activate the lymphocytes.

The present invention also provides a method for manufacturing a formula for preventing and treating tumors, various infectious diseases and autoimmune diseases, which comprises a step in which mononucleocytes are separated from peripheral blood containing HLA matching donor-originating lymphocytes, a step in which the HLA matching donor-originating lymphocytes in the separated mononucleocytes are propagated and activated and a step in which the lymphocytes having been propagated and activated are extracted and a formula containing them as a main constituent thereof is prepared.

Specific details of the present invention are explained in sequence below.

### (Type of peripheral blood to be collected)

According to the present invention, the peripheral blood that is collected needs to be peripheral blood originating from an HLA matching donor and, more desirably, peripheral blood originating from an HLA matching donor manifesting micro-chimerism. While it is desirable that the peripheral blood to be collected achieve a perfect HLA match with the HLAs of the patient so as to ensure that no GVHD-induced side effects occur, peripheral blood achieving a partial HLA match may be collected, instead.

For instance, peripheral blood originating from a donor with 1, 2, 3 or 4 loci among the 4 loci A, B, C and DR achieving a match with the corresponding loci of the patient can be used. Alternatively, peripheral blood of a donor with at least two HLA genes among the 8 HLA genes manifesting in the four loci achieving a match with those of the patient can be used. However, it is more desirable that four HLA genes achieve a match in this case.

An addition, it is also effective to use HLA matching donor-originating activated lymphocytes originating from a donor manifesting micro-chimerism. While micro-chimerism is a concept well established in the field of biomedicine, a brief explanation thereof is given below. "Micro-chimerism" (a state in which cells originating from another person are present in the body at a rate of approximately 1/1000) occurs in pregnancy, in which the mother and fetus exchange small numbers of cells with each other via the placenta.

In reality, micro-chimerism is invariably detected in a pregnant woman. In addition, even after the delivery, a great number of mothers sustain micro-chimerism over an extended period of time and thus, immunological tolerance to the child's HLAs originating from the father is sustained. Through this phenomenon, an immunological tolerance triangle is formed by the mother and her children.

Accordingly, the inventors of the present invention have confirmed through their research that activated lymphocytes originating from an HLA matching donor who is a sibling with micro-chimerism are highly effective in preventing and treating tumors, infectious diseases and autoimmune diseases. In addition, it has been revealed that peripheral blood can be used with a higher degree of safety even when the HLA genes do not achieve a match as long as it manifests micro-chimerism.

This means that as long as micro-chimerism is achieved, peripheral blood originating from a stranger with no blood relation can be used as effectively as peripheral blood originating from a parent, a grandmother, a grandfather, a sibling, an uncle, an aunt, a child or a grandchild. It is to be noted that each half of the HLA makeup of a child who genetically inherits the HLAs of the parents corresponds to the HLAs of one of the parents, and that his HLAs have a 25% chance of achieving a match with those of his sibling. The chance of his HLAs achieving such a match with a stranger having no blood relation are one in several hundred ∼ several tens of thousands, and for this reason, lymphocytes originating from a stranger cannot be used under normal circumstances. However, if a donor achieves micro-chimerism, the donor's lymphocytes can be used as material for the formula.

Furthermore, it is desirable to select a donor with whom the recipient achieves an HLA combination that works along the rejective direction. For instance, in an HLA combination that works along the rejective direction, the donor's A locus may include A2 and A24 sera and the A locus of the patient receiving the activated lymphocytes may include A2 and A2 sera. Since the patient's lymphocytes are capable of eliminating the donor's lymphocytes with the A24 serum, serious side effects such as GVHD can be avoided. An HLA combination that works along the rejective direction can be achieved with regard to the B, C and DR loci as well as with regard to the A locus.

### (Harvesting lymphocytes)

The donor-originating activated lymphocytes according to the present invention can be prepared from peripheral blood of the donor. While it is simple and therefore desirable to collect blood from a vein in the arm of the donor, no specific restrictions are imposed with regard to the collection site as long as the blood contains lymphocytes. Alternatively, lymphocytes collected from umbilical cord blood may be used. Only a small quantity of peripheral blood needs to be collected; in a range between 0.001 ml and 500 ml, and it is desirable to collect the peripheral blood in a quantity within a range of approximately 10 ml ∼ 100 ml in practical application.

### (Adjustment of lymphocytes)

Heparin or citric acid may be added into the collected peripheral blood so as to prevent coagulation. The donor-originating activated lymphocytes according to the present invention can be obtained by using the collected peripheral blood as a base material, preparing lymphocytes from the peripheral blood and then propagating and activating the lymphocytes through incubation. In addition, the lymphocytes according to the present invention can be prepared from the bone marrow of a donor through pheresis or the like. It is to be noted that in the explanation of the present invention, the term "incubation" of the harvested lymphocytes refers to an artificial propagation (incubation) of tissue cells achieved by placing a small sample of cell tissue in a culture solution containing necessary nutrients for the cells to survive and the term "propagation" refers to an artificial quantitative increase in the tissue cells achieved in vitro through the incubation described above. In addition, the term "activation" refers to control implemented to activate a stagnant function by stimulating the cells with the addition of a propagating agent or an activating agent into the culture solution and, more specifically, control implemented to achieve antineoplastic, antiviral and anti-autoimmune disease properties in the cells.

### (Propagation and activation of peripheral blood originating lymphocytes)

The activated lymphocytes originating from the collected peripheral blood can be propagated through any of the methods of lymphocyte incubation in the known art. While no specific restrictions are imposed with regard to the incubation method that may be adopted, the incubation may be achieved by, for instance, using either interleukin 2 or anti-CD3 antibodies alone or by using them in combination, as disclosed in Japanese Unexamined Patent Publication No. H 3-80076. It is desirable to incubate the lymphocytes in an environment in which both interleukin 2 and anti-CD3 antibodies are present, in order to achieve better propagation efficiency.

Instead of propagating and activating lymphocytes through polyclonal propagation / activation achieved by using interleukin 2 and anti-CD3 antibodies described above, activated lymphocytes achieving antigenic specificity can be obtained by first deriving antigen specific T-lymphocytes or the like with an appropriate antigen or the like as described earlier, and then by using one of; CD3 antibodies, CD26 antibodies, various mitogens and a cytokine such as interleukin 2 alone or by using them in combination.

Or activated lymphocytes achieving antigenic specificity may be selected by screening the activated lymphocytes achieved in the embodiment, activated lymphocytes achieving antigenic specificity may be derived by using an appropriate antigen, or activated lymphocytes achieving antigenic specificity may be prepared for use by propagating and activating the lymphocytes through antigen stimulation. In such a case, antigen specific lymphocytes can be derived either from CD8 positive cells or CD4 positive cells.

In addition to a refined antigen, an extract from a cancer cell or a virus, the cancer cell itself, the virus itself or an antigenic material manifesting cross reactivity to the cancer cell or the virus may be used for these purposes. Any of the substances described above may be used in this process as long as it has a function of propagating and activating lymphocytes. In addition, the interleukin 2 used in the process is a commercially available product and it should be dissolved so as to achieve a 1 ∼ 2000U/ml concentration in the incubating medium solution.

The interleukin 2 can be dissolved and used in any medium solution widely used for cell incubation, such as water, physiological saline solution, Dulbecco's phosphate buffer solution, RPMI-1640, DMEM, IMDM, and AIM-V. Once the interleukin 2 is dissolved, the solution should be refrigerated for storage so as to ensure that its activity is not reduced. It is to be noted that no specific restrictions are imposed with regard the type of incubating medium solution used for this purpose as long as it is suited for the incubation of lymphocytes, and an organism-originating culture solution such as a serum or a synthetic medium achieved by adding amino acids, vitamins, nucleic acid base and the like into a balanced saline, for instance, may be used. Desirable examples of the incubating medium solution include RPMI-1640, AIM-V, DMEM and IMDM, and among these, RPMI-1640 is particularly desirable.

While it is desirable to use an incubating medium containing normal human serum for better propagation, a commercially available medium product may be used as well. In addition, instead of the human serum: a serum originating from a bovine fetus may be used. Alternatively, a serum-free medium may be used. The incubation can be achieved by adopting any of the generally practiced incubation methods, e.g., inside a CO₂ incubator. In this case, it is desirable to sustain CO₂ concentration within a 1 ∼ 10% range and, more desirably, within a 3 ∼ 7% range, with the temperature sustained within a 30 ∼ 40°C range and, more desirably, within a 35 ∼ 38°C range.

While no specific restrictions are imposed with regard to the number of days required for the duration of incubation, it is a prerequisite that the incubation period be long enough to allow the stimulus information from anti-CD3 antibodies to be communicated to the cells. Accordingly, it is desirable to allow the incubation process to continue over approximately 2 ∼ 20 days, and it is particularly ideal to allow a stable stimulus information communication to the cells while achieving better incubation efficiency by incubating lymphocytes over a 3 ∼ 14 day period. It is even more desirable to ensure a higher degree of incubation efficiency during the incubation period by conducting microscopic observation of the cells, measuring the number of cells as necessary and replenishing the culture solution as necessary.

It is to be noted that while no marked increase in the number of cells is observed for the initial 1 ∼ 2 days after the incubation start, cell propagation is first observed around the third day and once the cells start to propagate in earnest, the culture solution changes color from orange to yellow. In addition, the culture solution should be replenished at a rate of approximately 10% ∼ 500% relative to the quantity of the medium solution prior to replenishment. Furthermore, the culture solution should be replenished once every 1 ∼ 7 days and, more desirably, once every 3 ∼ 5 days so as to prevent any degradation of the culture solution and to sustain the level of interleukin 2 activity.

Moreover, after the incubation in an environment in which anti-CD3 antibodies are present is completed, the incubation process may be allowed to continue without any stimulation from anti-CD3 antibodies. Namely, the incubation may be allowed to continue until the administration of the lymphocytes by using a tool in which anti-CD3 antibodies are not in solid phase, e.g., an incubating flask, a roller bottle, an incubating gas permeable bag.

When continuously incubating the lymphocytes as described above, the incubation should be allowed to progress under conditions which are the same as those set for the initial incubation under the influence of anti-CD3 antibodies, except that the second incubation takes place without any stimulus from anti-CD3 antibodies. It is more desirable to adjust the concentration of the human serum or to use a serum-free medium as necessary, in order to achieve better operability, better cost performance and better safety.

The incubation may be started by placing an incubation medium solution containing interleukin 2 with the umbilical cord blood or mononucleocytes suspended therein into an incubation container in which anti-CD3 antibodies are in solid phase. In this case, by adding various cytokines or mitogens into the culture solution as necessary, the efficiency with which the lymphocytes are propagated and activated is further improved. It is to be noted that while anti-CD3 antibodies used to stimulate the lymphocyte cells may be produced in an animal or in cells by using refined CD3 molecules, commercially available OKT-3 antibodies which achieve outstanding stability and cost performance (manufacturer: Orthopharmaceutical) may be used.

However, no restrictions are imposed with regard to the type of antibodies used to stimulate the lymphocytes as long as the antibodies is capable of promoting the propagation and the activation of lymphocytes, and the anti-CD20 antibody, for instance, may be used instead of anti-CD3 antibodies. In addition, it is desirable to use solid-phase anti-CD3 antibodies in order to achieve better lymphocyte propagation efficiency and better operability. The antibodies may be in solid phase in an incubation container constituted of glass, polyurethane, polyolefine or polystyrene. A commercial sterilized cell cultivating flask constituted of plastic or the like, which is readily available may be used for these purposes and, in such a case, the size of the flask can be selected as appropriate.

In addition, the antibodies can be induced into solid phase by adding a diluent of anti-CD3 antibodies into the container used for the purpose of processing the antibodies into solid phase and then leaving the container in a stationary state for 2 ∼ 24 hours with the temperature set to 4 ∼ 37°C. It is desirable that when processing anti-CD3 antibodies into solid phase, anti-CD3 antibodies be diluted to a concentration of 0.1 ∼ 30µg / ml in a physiological buffer solution such as sterilized Dulbecco's phosphate buffer solution. After the solid phase is achieved, anti-CD3 antibodies may be stored in a cold room or in a refrigerator (4 °C) until it is used and, in such a case, the liquid can be removed at the time of use and anti-CD3 antibodies can be readied for use by washing them with a physiological buffer solution such as Dulbecco's phosphate buffer solution at room temperature.

The donor peripheral blood originating activated lymphocytes thus obtained can be processed or prepared as a formula to be used as a means for biophylaxis over a wide range of applications, in preventing or treating tumors and various infectious diseases. In addition, the donor peripheral blood originating activated lymphocytes, which contain CD4 positive cells and CD8 positive cells, are expected to provide a better antineoplastic effect.

Furthermore, if there is a risk of any side effects such as GVHD, the prepared peripheral blood originating activated lymphocytes may be processed by using the antiCD4 antibodies or the like so as to prepare a cell population whose main constituent is CD4 positive cells to be used in the treatment. When the risk of side effects such as GVHD is low or when a higher antineoplastic effect is needed, on the other hand, the peripheral blood originating activated lymphocytes containing the CD8 positive cells can be directly used in the treatment. In the latter case, the activated lymphocytes can be used in the treatment as a cell population in which the CD8 positive cell content adjusted to the appropriate level.

### (Formula having peripheral blood originating activated lymphocytes as its main constituent)

The HLA matching donor-originating activated lymphocytes obtained from peripheral blood, which contains as its main constituent propagated peripheral blood cells, may be prepared as a formula by suspending them in, for instance, a physiological saline solution containing human albumin used as an infusion solution, and the formula thus prepared can be used in prevention and treatment of tumors and various infectious diseases. It is to be noted that the term "formula" as referred to in this context covers all materials achieving a biophylaxic function, which contain as their main constituent the activated lymphocytes, and such a formula may assume any mode as long as it contains the HLA matching donor-originating activated lymphocytes obtained from the donor's peripheral blood.

For instance, the formula may be provided in a form achieved by suspending the peripheral blood originating HLA matching activated lymphocytes in an appropriate solution, and while it is particularly desirable to provide the formula in a form in which the peripheral blood originating activated lymphocytes are suspended in a physiological saline solution containing human albumin, which is used as an infusion solution, as described above, the present invention is not limited to this example. In addition, the formula containing as its main constituent the peripheral blood originating activated lymphocytes may be prepared by directly propagating the peripheral blood within a test-tube or the formula containing as its main constituent the peripheral blood originating activated lymphocytes may be produced by first separating mononucleocytes from the peripheral blood and then propagating the separated mononucleocytes in a test tube.

Furthermore, various genes may be introduced into the peripheral blood originating activated lymphocytes contained in the formula according to the present invention, or any of the genes originally contained in the activated lymphocytes may be deleted or modified. Peripheral blood originating activated lymphocytes from which the cofactor that allows a human immunodeficiency viral infection is deleted can be used to prevent or treat diseases of human immune deficiency caused by viral infection or to prevent or treat various infections occurring in a state of immunodeficiency caused by the human immunodeficiency virus.

The formula containing as its main constituent the HLA matching donor-originating activated lymphocytes harvested from the peripheral blood as described above can be used in a wide range of clinical applications to prevent and treat cancer patients, patients with immune deficiency, patients with autoimmune diseases, patients with diseases requiring umbilical cord blood infusions such as allergy patients and patients with various types of infections. The formula according to the present invention can be used to treat tumors as well as to prevent recurrence of tumors, and it can also be used in the treatment of leukemia and various types solid tumors.

Moreover, by using the formula to prevent or treat a tumor as described above, the QOL (quality of life) can be improved for the patient as well. Namely, if, for instance, an anti-cancer drug causes a lingering kidney or liver disorder or the patient is left with a permanent scar from surgery, such a side effect or the like of the treatment often compromises the quality of life of the patient even after he is cured of the disease itself. However, by treating the patient with the formula according to the present invention, his QOL can be greatly improved.

The formula containing as its main constituent the HLA matching donor-originating activated lymphocytes according to the present invention is effective in treating various types of cancer and tumors, immunodeficiency disorders and various types of infections as detailed later. In more specific terms, it is effective in treating lung cancer, stomach cancer, colon cancer, rectal cancer, kidney cancer, pancreatic cancer, gallbladder cancer, ovarian cancer, cancer of the uterus, testicular cancer, prostate cancer, leukemia, sarcoma and brain tumor. It is also effective in treating both congenital and acquired immunodeficiency.

While the congenital immunodeficiency diseases include severe combined immunodeficiency, Wiscott Aldrich syndrome, Adenosine deaminase deficiency, purine nucleoside phosphorylase deficiency, high IgM syndrome and combined immunodeficiency, the formula according to the present invention may be used to treat an immunodeficiency other than those. In addition, while typical examples of acquired immune deficiency include secondary immunodeficiency caused by the use of anti-cancer drugs, immunosuppressive agents or steroids and AIDS caused by human immunodeficiency viral infection, the present invention may be used to treat an immunodeficiency other than these as well.

In addition, while typical examples of autoimmune diseases that may be treated with the formula according to the present invention include systematic lupus erythematosus, chronic articular rheumatism, Sjögren's syndrome, myasthenia gravis, pernicious anemia and Hashimoto's disease, the present invention may be used to treat other autoimmune diseases as well. The formula according to the present invention may be used as preventative or therapeutic measures in treating a patient with no immunological competence or a low degree of immunological competence against a specific virus or the like, as well as in treating a patient with generally poor immunological competence. While the allergic diseases that may be treated by using the formula according to the present invention include bronchial asthma, cedar pollen allergies and urticaria, the formula according to the present invention may be used to treat allergies other than those and also it may be used to lessen the symptoms of various allergies.

The infectious diseases that may be treated with the formula according to the present invention include viral infections, microbisms, fungal infections, protozoan infections, chlamydia infection and mycoplasma infections. Of those, the viral infections include cytomegalovirus infection and Epstein-Barr virus infection. However, the application of the present invention is not limited to the treatment of these viral infections, and the formula according to the present invention can be used in a wide range of applications in preventing and treating various other viral infections such as herpes, including herpes simplex virus and Varicella-Zoster virus, various retroviruses including the human leukemia viruses and human immunodeficiency viruses, adenoviruses and coxsackie viruses.

The microbisms that may be treated with the formula according to the present invention include infections caused by pseudomonas aeruginosa and methicillin-resistant yellow staphylococcus. However, the formula according to the present invention can be used to treat any infectious disease caused by a bacterium manifesting pathogenicity in humans. In particular, the formula according to the present invention can be used effectively in preventing and treating infectious diseases caused by unknown pathogens, pathogens that cannot be identified easily or in preventing and treating diseases resulting from such infections.

The micro-chimerism described earlier can be easily detected through PCR (polymerase chain reaction) analysis. For instance, when measuring micro-chimerism of A-24 in the HLA-A locus, the presence/absence of micro-chimerism can be determined by first amplifying the reaction with an HLA-A locus primer, executing a nested-PCR with an A-24 primer and then detecting any micro-chimerism with an appropriate detection system.

### (Preparation kit to be used to prepare formula containing as its main constituent peripheral blood originating activated lymphocytes)

While the constituents of HLA matching donor-originating activated lymphocytes according to the present invention can be used as a single reagent preparation, they may be prepared in a kit by combining a culture solution containing interleukin 2 and solid-phase anti-CD3 antibodies in a flask as well. In the latter case, the formula according to the present invention can be prepared with even greater ease by using the kit.

It is to be noted that the culture solution used to prepare the formula may be poured in appropriate quantities in advance into separate flasks coated with solid-phase anti-CD3 antibodies and then these flasks may be stored in a freezer. By preparing the activated lymphocytes in a kit achieved by combining at least two components as a plurality of reagents as described above, the kit can be used when the need for activated lymphocytes has risen to prepare the formula according to the present invention with greater ease. The HLA matching donor-originating activated lymphocytes prepared from peripheral blood or the formula containing such activated lymphocytes, which may be used in all modes of the present invention described above, can be stored in a freezer so as to be ready for use in the prevention or treatment of various diseases whenever necessary.

### (Dosage)

While the dose of the formula containing as its main constituent the HLA matching activated lymphocytes prepared from peripheral blood should be adjusted as appropriate in accordance with the state of the specific patient or the specific objective of the treatment, the standard dose administered under normal circumstances varies between 1 × 10² to 1 × 10⁹ lymphocytes relative to 1 kg of body weight. In addition, it is desirable to administer 1 × 10³ lymphocytes or more per kg in order to further enhance the effect, but, at the same time, the effect does not increase any more once the dose exceeds 5 × 10⁸ lymphocytes per kg. Thus, the optimal range of dose is 1 × 10³ ∼ 5 × 10⁸ lymphocytes per kg.

### (Modes and methods of administration)

The formula described above should be administered in liquid form, i.e., in an injection or in a drip. More specifically, it is desirable to administer the cells prepared as described above in an injection or a drip containing the cells dispersed in a physiological saline solution in which human blood serum albumin is added to a concentration of 0.01 ∼ 5%. The formula should be administered in an intravenous drip or in an intravenous injection, an arterial injection or a topical injection. While the quantity of solution that is administered needs to be adjusted depending upon the administration method, the administration site or the like, it is desirable to keep the quantity of the solution being administered within the range of 1 ∼ 500 ml under normal circumstances and, at the same time, to ensure that the required number of lymphocyte cells mentioned earlier is contained in the solution being administered. In addition, the frequency of such administration should be once a day ∼ once a month, and the lymphocytes should be administered at least more than once.

### (Embodiment 1)

### No. 1. (Blood collection and separation of lymphocytes)

20 ml of peripheral blood of a donor achieving a complete HLA match with an acute lymphatic leukemia patient was collected from the vein by adding heparin with an injection syringe. The injection needle was then disengaged from the injection syringe while maintaining aseptic conditions within a clean bench and the injection needle was replaced with a 19 G × 11/2" injection needle. 15 ml of a washing medium (RPMI 1640+6) (500 ml, manufacturer: Nikken Bio-medical Research Center, GM1106) had been poured into each of two 50 ml centrifugation tubes (manufacturer: Iwaki Glass Co. Ltd., 2341-050), and after diluting the collected blood with a culture solution to increase the quantity of the solution by a factor of 3, the entire mixture was slowly poured into the two centrifugation tubes so that exactly equal quantities of the mixture would be poured into the two tubes.

After completely closing the lids of the centrifugation tubes, they were turned and agitated two or three times. 3 ml of Lymphocepar 1 (100 ml, manufacturer: Immunobiological Bio-research Center Co. Ltd., 23010) was placed into each of six 15 ml centrifugation tubes with a 10 ml pipette, and then, 10 ml of the blood having been diluted with the medium was slowly stratified over the Lymphocepar 1 so as not to disturb the surface in each centrifugation tube. The centrifugation tubes were then centrifuged for 15 minutes in a centrifuge at 1800 rpm while maintaining a centrifuging temperature of 20°C.

When the centrifugation was completed, the contents of each centrifugation tube was slowly suctioned down to approximately 1 cm above the lymphocyte layer with an aspirator so as not to suction off the lymphocyte cells, while maintaining aseptic conditions. Then, using a 5 ml Pipetman, the lymphocyte cell layer drawn off without suctioning off the blood clot layer, and the lymphocyte cell layer thus extracted was collected into a 50 ml centrifugation tube into which 25 ml of the washing medium (RPMI 1640+6) had been placed in advance. After this centrifugation tube was removed and mixed gently by inversion with its lid closed, the centrifugation tube was placed in the centrifuge again to undergo centrifugation for 10 minutes at 1800 rpm with the centrifuging temperature set to 20°C.

After the centrifugation, the supernatant fluid was discarded and the cell sediment was thoroughly loosened and stirred. After measuring the number of cells, the cells were placed in a suspension in PBS at a concentration of 0.5 × 10⁶/ml. After the cells were mixed with Dynabeads CD4 (importer/vendor: Veritas) at a cell/bead ratio of 1:4 in a 15 ml-capacity centrifugation tube, the mixture was incubated for 30 minutes at 4°C. The CD4 positive cells bonded with Dynabeads CD4 were collected by placing a magnet (importer/vendor: Veritas) in contact with the outer wall of the centrifugation tube, whereas cells which had remained unbonded with any Dynabeads CD4 were aseptically suctioned off by using an aspirator.

The cells bonded with the Dynabeads CD4 were then thoroughly loosened in a vortex. Then, they were mixed gently by inversion in 50 ml of an incubation medium achieved by infusing 1 ml of 3500U/ml IL-2 (manufacturer: Cetus) and 5 ml of human blood serum in 44 ml of a medium (RPMI 1640 + 7 manufacturer: Immunological Bio-research Center Co. Ltd.) and thus, a cell suspension was prepared.

10µl of the cell suspension was taken into a tube (importer/vendor: Assist Co. Ltd., 72.690) and then the suspension was blended with 40µl of Türk solution. 10µl of the mixture was placed on a hemocytometer (manufacturer: Elmer Inc., 9731) and the number of cells was measured under a microscope. The total cell count was 1.0 ×10⁷ ∼ 7.0 × 10⁷.

### No. 2 (Preparation of OKT3-coated flask)

10 ml of OKT3 solution (importer/vendor: Jansen Kyowa, Co. Ltd., manufacturer: Orthopharmaceutical: OKT3 injection) having been adjusted to achieve a 5µg/ml concentration with PBS (-) was placed into an incubation flask having a base area of 225cm² by ensuring the bottom surface of the flask was evenly covered with the solution.

The following day, the OKT3 solution in the flask was suctioned off with an aspirator, and then, 50 ml of PBS (-) was poured into the flask. After the flask was agitated thoroughly with its lid closed, the lid was opened and the liquid was discarded. Next, 50 ml of PBS (-) was poured into the flask while sustaining an aseptic state, then the flask was thoroughly agitated with the lid closed. The lid was then opened and the liquid was discarded. Any moisture remaining inside the flask or on the lid was suctioned off thoroughly with the aspirator and thus, an OKT3-coated flask was prepared.

### No.3 (Activating incubation of lymphocytes)

50 ml of the cell suspension prepared as described in the above No. 1 (Blood collection and separation of lymphocytes) was poured into each OKT3-coated flask prepared as described in the above No. 2 (Preparation of OKT3-coated flask) and then the suspension was incubated in the flask at 37°C in an environment in which carbon dioxide gas with a 5% concentration was present. Three days later, 50 ml of the incubation medium was added and the incubation was carried on at 37°C in the environment in which the carbon dioxide gas with a 5% concentration was present. Then, four days later, 150 ml of the incubation medium was added and a the incubation was carried on at 37°C in the environment in which the carbon dioxide gas with a 5% concentration was present. After the final replenishment of the incubation medium, the incubation was allowed to last for another two days at 37°C in the environment in which the carbon dioxide gas with a 5% concentration was present. As a result, 2.0 × 10⁸ ∼ 7.0 × 10⁸ activated lymphocytes were obtained.

### No.4 (Propagating incubation of lymphocytes)

The lymphocytes prepared through the above No. 3 (Activating incubation of lymphocytes) above were transferred into a gas permeable incubation bag containing 750 ml of an LL-7 medium (Nikken Bio-medical Research Center) and then the lymphocytes thus transferred were incubated inside a carbon dioxide gas incubator (CDP-300A; Hirasawa Co. Ltd.) at 37°C within a 5% carbon dioxide gas atmosphere.

Four days later, the gas permeable incubation bag containing the cells (manufacturer: Nipro Co. Ltd., Nipro culture bag A-1000) and another gas permeable incubation bag containing a new medium were joined by using an aseptic conjugation device (manufacturer: Terumo) and the media inside the two gas permeable incubation bags were thoroughly mixed. Then, the connection between the bags was cut and after the areas of the junction were aseptically sealed, the cells were incubated at 37°C in a 5% carbon dioxide gas atmosphere.

### No.5 (Preparation of formula for administration)

Three days later, the medium containing the cells in the two gas permeable bags was transferred into a centrifugation tube with a capacity of 250 ml and the cells were separated through centrifugation. Then, the culture solution was eliminated through decantation, a physiological a saline solution was added to the cell pellets so as to re-suspend the cells and then the cells were washed through centrifugation. Next, a similar washing operation was executed by using a physiological saline solution containing human albumin at a concentration of 0.1% instead of the physiological saline solution used earlier and thus, the cell pellets were adjusted.

Next, 200 ml of physiological saline solution containing human albumin at 2% concentration was added to the cell pellets to suspend the cells and, after the suspension was filtered through a 100µm stainless steel mesh, the preparation was packed into a transfusion bag and was readied for administration. It is to be noted that the number of cells transferred into the transfusion bag was 0.5 ∼ 10 × 10⁹.

### 6. (Freezer storage of formula)

It is to be noted that the activated lymphocytes prepared in the above No. 5 (Preparation of formula for administration) as described above may be stored in a freezer as necessary. To explain a specific embodiment in which the formula is preserved in a freezer, the activated lymphocytes obtained in the above No. 5 (Preparation of formula for administration) were separated through centrifugation, the incubation medium was removed through decantation thereby obtaining cell pellets, 18 ml of a cell preserving solution (prepared by mixing 5 ml of human blood serum, 5 ml of dimethyl sulfoxide (manufacturer: Nakaraitesky Co. Ltd., it may be hereafter referred to as "DMSO") and 40 ml of a medium (RPMI 1640 + 7)) was added to the cell pellets, the cell pellets and the preserving solution were mixed thoroughly, and 3 ml of the mixture was poured into each of five 5 ml cell preserving tubes (5 × 10⁷ cells/tube). These tubes were then placed in an ultra low-temperature freezer and were preserved at -80°C.
In addition, the frozen cells should be thawed and liquefied for use by taking the frozen cells out of the freezer and warming them with a 37°C heat block (manufacturer: Titek Inc.; TAL-IG) for 4 minutes. We aseptically transferred approximately 3 ml of the cell preserving solution containing cells thus thawed and liquefied into a 15 ml centrifugation tube, suspended the cells by adding 10 ml of a culture solution and then separated the cells through centrifugation (executed at 1000 rpm at 20°C over 5 minutes). Afterwards, the supernatant fluid was discarded through decantation, the cells were incubated through the method of described in 1 ∼ 4 above and the formula for administration was prepared as described in the above No, 5 (Preparation of formula for administration) above. We also prepared the formula ready for administration by washing the thawed and liquefied cells with an incubation physiological saline solution and then adding 10 ml of a physiological saline solution containing human blood serum albumin at 5% concentration to allow them to become suspended.

### No. 7 (Administration of formula)

The formula prepared as described in the above No. 5 (Preparation of formula for administration) or preserved in the freezer as described in the above No. 6 (Freezer storage of formula) was injected intravenously into an acute lymphatic leukemia patient who had not undergone hematopoietic stem cell transplant. The acute leukemia patient who had demonstrated resistance to various chemotherapy drugs and had not gone into remission in spite of the chemotherapy was administered the formula a total of 30 times. 1.7 × 10⁸ ∼ 4.2 × 10⁹ cells were injected at a time every few days to every 12 days (usually, every 7 days).

### No. 8 (Evaluation of effects)

While the various chemotherapies that had been tried prior to the administration of the HLA matching donor-originating activated lymphocytes had all proved ineffective and an increase in leukemic cells had been observed, the following was recorded following the administration of the HLA matching donor-originating activated lymphocytes:
(1) The leukemic cells did not increase markedly. Rather, a tendency for a gradual decrease was observed (antineoplastic effect);
(2) The patient did not contract any serious infections and during the treatment and an increase in neutrophillic leukocytes was observed (increased at a rate of 500/µl or greater following the administration) (effect against infectious disease); and
(3) During the initial stage of the treatment, the pain was reduced (improvement of QOL and effect against autoimmune disease).

Thus, it has become clear that the HLA matching donor-originating activated lymphocytes are effective preventive and a therapeutic means against tumors, infectious diseases and autoimmune diseases and are also effective in improving the quality of life of the patient. In addition, hardly any side effects of the HLA matching donor-originating activated lymphocytes administration were observed, and no GVHD symptoms or high temperature was observed.

### (Embodiment 2)

Activated lymphocytes were prepared as in embodiment 1 except that peripheral blood was collected from a blood relative (with two HLA loci achieving a match with the patient) manifesting microchimerism with an acute myelocytic leukemia patient. The activated lymphocytes were prepared as a formula ready for administration as in embodiment 1, and this formula was injected through a vein into the acute myelocytic leukemia patient not having undergone a hematopoietic stem cell transplant. The formula was administered only once. Three days after the administration, a decrease in the leukemic cells in the patient's peripheral blood was observed, and the leukemic cells in the peripheral blood disappeared altogether seven days after the administration. Thus, it became clear that activated lymphocytes originating from a donor achieving microchimerism has an antineoplastic effect.

### (Embodiment 3)

Activated lymphocytes were prepared as in embodiment 1 except that peripheral blood was collected from a blood relative (with two HLA loci achieving a match with the patient) manifesting microchimerism with a kidney cancer patient. The activated lymphocytes were prepared as a formula ready for administration as in embodiment 1, and this formula was injected through a vein into the kidney cancer patient. The formula was administered every month for a total of 3 times. Two weeks after the initial administration, a partial reduction in the kidney cancer was observed, and the kidney cancer disappeared altogether two weeks after the third administration. Thus, it became clear that activated lymphocytes originating from a donor achieving microchimerism has an antineoplastic effect.

As has been proved through the embodiments described above, activated lymphocytes highly effective as preventive and therapeutic means against tumors, viral infections and autoimmune diseases can be obtained by collecting and activating lymphocytes of an HLA matching donor, and then stimulating and propagating these activated lymphocytes with interleukin 2 and anti-CD3 antibodies or the like. It has also been clearly demonstrated that the HLA matching donor-originating activated lymphocytes effective in improving the QOL of the patient as well as being effective against tumors, infectious diseases and autoimmune diseases, are highly useful as preventive and therapeutic means against tumors, infectious diseases and autoimmune diseases.

In addition, the HLA matching donor-originating activated lymphocytes have hardly any adverse side effects, and no manifestation of GVHD symptoms or high-temperature has been observed.

Furthermore, the present invention can be adopted for a specific purpose of improving the quality of life for the patient by lessening the malaise and increasing the patient's appetite as well as for purposes of treating tumors such as cancer and various infectious diseases, preventing the recurrence of tumors and infectious diseases and preventing tumors and infectious diseases. The formula according to the present invention can be used as a preventive and therapeutic means for treating various solid tumors as well as leukemia.

In particular, the advantages of the present invention can be further enhanced by treating the patient with a combination of the present invention and another therapeutic method such as chemotherapy or radiation therapy or with a combination of the present invention and another immuno-therapy. Moreover, the present invention may be adopted for the purpose of improving the state of the patient prior to an hematopoietic stem cell transplant.

In addition, the stability and efficiency of the lymphocyte activation can be greatly improved by using at least either interleukin 2 or anti-CD3 antibodies to activate the HLA matching donor-originating activated lymphocytes, by using one of; CD3 antibodies, CD26 antibodies or a cytokine such as interleukin 2 or by using them in combination to obtain antigen specific activated lymphocytes after deriving T-lymphocytes or the like achieving antigenic specificity with an appropriate antigen or the like or by propagating and activating antigen specific lymphocytes derived from CD8 positive cells or CD4 positive cells with an antigen stimulus to prepare activated lymphocytes achieving antigenic specificity. Then, a formula containing the activated lymphocytes can be prepared, readily available and convenient for administration to be used as a preventive and therapeutic means against tumors, various infectious diseases and autoimmune diseases. In this sense, the present invention is expected to greatly contribute to a further development in the field of regenerative medicine.

By preparing a formula of the propagated and activated lymphocytes in the form of a suspension in which the lymphocytes are suspended in an infusion physiological saline solution containing human albumin, the extent to which the number of effective activated lymphocytes becomes lower at the time of administration can be reduced.

In addition, the risk of GVHD can be further lowered by ensuring that the HLA matching donor-originating activated lymphocytes being administered contain HLAs that work along the rejective direction.

By using lymphocytes collected from a donor achieving micro-chimerism, even more effective activated lymphocytes for preventing and treating tumors, infectious diseases and autoimmune diseases can be obtained and, at the same time, the occurrence of side effects such as GVHD can be reduced. These advantages can become even more marked by using activated lymphocytes containing CD8 positive cells as well as CD4 positive cells.

By marketing the HLA matching donor-originating activated lymphocytes as a preparation kit to be used to prepare activated lymphocytes constituted of HLA matching donor-originating lymphocytes and at least interleukin 2, which activates the lymphocytes, or to be used to prepare a formula containing the HLA matching donor-originating activated lymphocytes, the HLA matching donor-originating activated lymphocytes according to the present invention can be made readily available even at remote locations, and HLA matching donor-originating lymphocytes can be propagated and activated to be used in a therapy for preventing and treating tumors, various infectious diseases and autoimmune diseases without requiring any advanced knowledge or extensive experience.

The method for manufacturing a formula containing lymphocytes to be used to prevent and treat tumors, various infectious diseases and autoimmune diseases comprises a step in which mononucleocytes are separated from peripheral blood containing HLA matching donor-originating lymphocytes, a step in which the HLA matching donor-originating lymphocytes in the separated mononucleocytes are activated and propagated by using, for instance, at least either interleukin 2 or anti-CD3 antibodies and a step in which the lymphocytes having been activated and propagated are extracted and are prepared as a formula containing the lymphocytes as a main constituent thereof. As a result, the formula containing the HLA matching donor-originating lymphocytes can be manufactured efficiently and, at the same time, it becomes possible to prevent any reduction in the level of activity and in the number of effective lymphocytes.

While propagating lymphocytes through incubation, the culture solution may be replenished at a rate of 10% ∼ 500% relative to the quantity of the incubation medium solution during the 3 ∼ 14 day incubation period, and such a replenishment may be effected once every 3 ∼ 5 days during the 3 ∼ 14 day incubation period to prevent any degradation of the culture solution and to prevent any reduction in the level of the activity of interleukin 2. Furthermore, by adding various cytokines or mitogens while activating and propagating the lymphocytes through incubation, the effect of the activated lymphocytes can be further enhanced.

Moreover, HLA matching donor-originating lymphocytes having been activated and propagated, which contain CD4 positive cells and CD8 positive cells prove even more effective than HLA matching donor-originating activated lymphocytes containing CD4 positive cells alone.

## Claims

1. Preventive and therapeutic lymphocytes for preventing and treating tumors, infectious diseases and autoimmune diseases, **characterized in that** said lymphocytes are obtained by activating HLA matching donor-originating lymphocytes.

2. Preventive and therapeutic lymphocytes according to claim 1, **characterized in that**:
said HLA matching donor-originating lymphocytes are activated with interleukin 2.

3. Preventive and therapeutic lymphocytes according to claim 1, **characterized in that**:
said HLA matching donor-originating lymphocytes are activated with anti-CD3 antibodies.

4. Preventive and therapeutic lymphocytes according to claim 1, 2 or 3, **characterized in that**:
said HLA matching donor-originating lymphocytes are antigen specific activated lymphocytes obtained by deriving antigen specific lymphocytes with an appropriate antigen and then by using one of; CD3 antibodies, CD26 antibodies and interleukin 2 or using CD3 antibodies, CD26 antibodies and interleukin 2 in combination.

5. Preventive and therapeutic lymphocytes according to any one of claim 1 through 4, **characterized in that**:
said HLA matching donor-originating lymphocytes are prepared and used by first activating and propagating HLA matching donor-originating lymphocytes which have been collected, and then by selecting through screening antigen specific activated lymphocytes or deriving antigen specific activated lymphocytes with an appropriate antigen.

6. Preventive and therapeutic lymphocytes according to any one of claim 1 through 4, **characterized in that**:
said HLA matching donor-originating lymphocytes are obtained by preparing antigen specific lymphocytes having been activated and propagated with antigen stimulation.

7. Preventive and therapeutic lymphocytes according to any one of claim 4 through 6, **characterized in that**:
said antigen is a refined antigen, an extract from a cancer cell or a virus, a cancer cell or a virus, or an antigenic material achieving cross reactivity to a cancer cell or a virus.

8. Preventive and therapeutic lymphocytes according to any one of claim 1 through 7, **characterized in that**:
said HLA matching donor-originating lymphocytes contain HLAs that work along a rejective direction.

9. Preventive and therapeutic lymphocytes according to any one of claim 1 through 7,**characterized in that**:
said HLA matching donor-originating lymphocytes are collected from a donor achieving micro-chimerism.

10. Preventive and therapeutic lymphocytes according to any one of claim 1 through 9, **characterized in that**:
a correct dose of said lymphocytes per kilogram of body weight is within a range of 1 × 10³ ∼5 x 10⁸.

11. A preventive and therapeutic formula used for preventing and treating tumors, various infectious diseases and autoimmune diseases, **characterized by** obtaining HLA matching donor-originating activated lymphocytes.

12. A preventive and therapeutic formula according to claim 11, **characterized by** being prepared by suspending lymphocytes having been activated and propagated in an infusion physiological saline solution containing human albumin.

13. A preventive and therapeutic formula according to claim 11 or 12, **characterized in that**:
a correct dose of said lymphocytes per kilogram of body weight is within a range of 1 × 10³ ∼5 x 10⁸.

14. A formula preparation kit, **characterized by** being constituted of a combination of HLA matching donor-originating lymphocytes and, at least interleukin 2 for activating said lymphocytes.

15. A formula preparation kit, **characterized by** being constituted of a combination of HLA matching donor-originating lymphocytes and, at least anti-CD3 antibodies for activating said lymphocytes.

16. A method for manufacturing a preventive and therapeutic formula for preventing and treating tumors, various infectious diseases and autoimmune diseases, **characterized by** comprising:
a step in which mononucleocytes are separated from peripheral blood containing HLA matching donor-originating lymphocytes;
a step in which said HLA matching donor-originating lymphocytes in said mononucleocytes having been separated are activated and propagated; and
a step in which said lymphocytes having been activated and propagated are extracted and prepared as a formula having said lymphocytes as a main constituent thereof.

17. A method for manufacturing a preventive and therapeutic formula for preventing and treating tumors, various infectious diseases and autoimmune diseases according to claim 16, **characterized in that**:
said lymphocytes are propagated over a 3 ∼ 14 day continuous incubation period by using an incubation medium combined with either interleukin 2 or anti-CD3 antibodies or combined with both interleukin 2 and anti-CD3 antibodies.

18. A method for manufacturing a preventive and therapeutic formula for preventing and treating tumors, various infectious diseases and autoimmune diseases according to claim 16 or 17, **characterized in that**:
a culture solution is added at a rate of 10% ∼ 500% relative to the quantity of the incubation medium solution during said 3 ∼ 14 day incubation period to prevent any degradation of said culture solution and prevent any decrease in the level of activity of interleukin 2 when propagating said lymphocytes through incubation.

19. A method for manufacturing a preventive and therapeutic formula for preventing and treating tumors, various infectious diseases and autoimmune diseases according to claim 18, **characterized in that**:
said culture solution is added once every 3 ∼ 5 days during said 3 ∼ 14 day incubation period.

20. A method for manufacturing a preventive and therapeutic formula for preventing and treating tumors, various infectious diseases and autoimmune diseases according to any one of claim 16 through 19, **characterized in that**:
various cytokines or mitogens are added into a culture solution when activating and propagating said of lymphocytes through incubation.

21. A method for manufacturing a preventive and therapeutic formula for preventing and treating tumors, various infectious diseases and autoimmune diseases according to claim 16, **characterized in that**:
said HLA matching donor-originating lymphocytes having been activated and propagated contain CD4 positive cells and CD8 positive cells.

22. A preventative and therapeutic method for preventing and treating patients with tumors, infectious diseases and autoimmune diseases **characterized by** administering lymphocytes according to claim 1 to said patients.

23. A preventative and therapeutic method according to claim 22, **characterized in that**:
said HLA matching donor-originating lymphocytes are activated with interleukin 2.

24. A preventative and therapeutic method according to claim 22, **characterized in that**:
said HLA matching donor-originating lymphocytes are activated with anti-CD3 antibodies.

25. A preventative and therapeutic method according to claim 22, 23, or 24, **characterized in that**:
said HLA matching donor-originating lymphocytes are antigen specific activated lymphocytes obtained by deriving antigen specific lymphocytes with an appropriate antigen and then by using one of; CD3 antibodies, CD26 antibodies and interleukin 2 or by using CD3 antibodies, CD26 antibodies and interleukin 2 in combination.

26. A preventative and therapeutic method according to any one of claim 22 through 25, **characterized in that**:
said HLA matching donor-originating lymphocytes are prepared and used by first activating and propagating HLA matching donor-originating lymphocytes which have been collected and then by selecting through screening antigen specific activated lymphocytes or deriving antigen specific activated lymphocytes with an appropriate antigen.

27. A preventative and therapeutic method according to any one of claim 22 through 26, **characterized in that**:
a correct dose of said lymphocytes per kilogram of body weight is within a range of 1 × 10³ ∼ 5 × 10⁸.
